# EUROPEAN PATENT APPLICATION

(11) **EP 1 357 131 A2**
(43) Date of publication of application: **29.10.2003**
(21) Application number: 03014404.2
(22) Date of filing: 31.12.1997
(51) Int. Cl.: C07K 16/28, A61K 39/395, A61P 13/12, A61P 37/00

(54) **Treatment of lupus nephritis with anti-CD4OL compounds**

(30) Priority: 10.01.1997 US 34673 P
(62) Divisional of application: 97953325.4
(71) Applicant: BIOGEN, INC., Cambridge Massachusetts 02142 (US)
(72) Inventor: Kalled, Susan L., Jamaica Plain, MA 02130 (US); Thomas, David W., Wellesley, MA 02182 (US)
(74) Representative: UEXKÜLL & STOLBERG

(57) **Abstract**

Methods are provided for treating nephritis associated with immune complex disease with anti-CD40L compounds. In one embodiment, anti-CD40L compounds are administered to a patient with immune complex disease who has received a kidney allograft, to inhibit the development of immune complex glomerulonephritis within the grafted kidney.

## Description

### Field of the Invention

The invention relates to administration of anti-CD40L compounds to patients for treatment of immune complex glomerulonephritis. and in particular, lupus nephritis.

The present invention relates to methods and compositions for the treatment of immune complex glomerulonephritis. More particularly, this invention relates to the use of compounds that bind to the ligand for the CD40 surface molecule of B and various other cells, alone or in combination with other agents, for treating or reducing the advancement, severity, symptoms or effects of nephritis associated with antibody-mediated disease, such as lupus or drug-induced serum sickness. According to one embodiment, this invention employs the monoclonal antibody 5c8.

### Background of the Invention

Immune complex disease is mediated by the deposition of immune complexes in certain tissues, including the renal glomerulus and blood vessel walls. The complexes are aggregates of antigen and antibodies. The antigens may be autoantigens. when the body produces antibodies against components of its own tissues, or exogenous antigens, such as infectious agents or drugs. In each case, deposits of immune complexes within blood vessels can cause skin eruptions, pericarditis, and vasculitis. Immune complex deposits within the glomerulus can interfere with the filtering capability of the kidney, leading in extreme cases to renal failure and death.

Systemic lupus erythematosus (SLE) is a life threatening autoimmune disease, characterized by the production of autoantibodies against various tissues, and often against DNA. SLE affects approximately 140.000 people in the United States and 105,000 in western Europe, predominantly women of childbearing age.

SLE is characterized by inflammation affecting connective tissues including skin. joints and organ systems; frequently affected organs include the kidneys, heart, lungs and central nervous system. Clinical manifestations of SLE often include generalized illness, pain. rash. cognitive dysfunction, thrombosis, anemia, pleurisy, gastrointestinal dysfunction, and fetal loss in pregnant women. In most patients, lupus-associated immunoglobulins and immune complexes are deposited in the renal glomeruli, causing a decline in renal function. Of SLE patients with moderate to severe disease, who comprise 70% of total lupus patients, half develop clinical nephritis, characterized by the presence of protein in the urine. While some of these patients can be successfully treated with immunosuppressive and/or cytotoxic drugs, the clinical response to these drugs may be transient, the drug therapy causes undesirable side effects, and many patients do not respond to the available pharmaceutical therapies. A substantial percentage of these patients progress to renal failure, and must then undergo repeated dialysis for life, or seek a kidney transplant. The transplant may itself be short-lived, as the new kidney is also susceptible to damage from the unremitting autoantibodies present in the blood of the SLE patient. The median age at which an SLE patient begins dialysis is 35. Many SLE patients eventually die as a direct or indirect result of nephritis.

Therapeutics used in the treatment of SLE fall into various classes: salicylates and non-steroidal anti-inflammatory agents, steroidal anti-inflammatory agents (systemic corticosteroids), immunosuppressive agents or cytotoxic drugs, antimalarial drugs, dialysis, transplant, lymphoid irradiation or plasmapheresis. The majority of these act as symptomatic agents, designed to ameliorate inflammation. targeting the symptoms of the disease and exerting effects both upon and throughout the total course of administration. Examples of symptomatic agents are salicylic acid, glucocorticoids and non-steroidal anti-inflammatory agents, such as indomethacin. Other therapeutics act on the pathogenesis of the disease to inhibit the autoimmune reactions, exerting their effects only after the initial weeks of administration, yet having effects lasting beyond the cessation of treatment. Such agents include immunosuppressants such as the chemotherapeutics azathioprine and cyclophosphamide, as well as cortisones such as prednisone.

The general immunosuppressants now used to treat SLE often cause adverse side effects, such as increasing the patient's susceptibility to infection, which contraindicate the long term use of these agents. The use of selective immunosuppressants which target specific steps in the pathogenesis of the disease have begun to be explored in attempts to reduce or normalize the immunoglobulin concentration in SLE patients; this would be expected to have beneficial effects on the renal manifestations of the disease. One of the necessary reactions in the generation of antibodies is the interaction of CD40 on B cells with CD40 ligand (CD40L) on activated T cells, a step which is required for B cell growth and subsequent production of antibodies. (Note: "gp39" is used synonymously for CD40L in some reports.)

Two groups have blocked the CD40-CD40L interaction with anti-CD40L monoclonal antibodies (mAbs) in young NZB cross mice, and examined the effects on the subsequent development of nephritis in the mice. (Mohan et al., J. Immunol. 154: 1470-1480, 1995; Early et al., J. Immunol. 157: 3159-3164, 1996) The female offspring of crossing New Zealand Black (NZB) mice either with New Zealand White (NZW) mice or with normal SWR mice [respectively called (NZB X NZW) F₁ and (SWR X NZB) F₁ ]develop a syndrome similar in many respects to SLE in humans (Steinberg et al., Ann. Int. Med. 115:548, 1991; Wofsy and Seaman, J. Exp. Med. 161:378, 1985). The female F₁ mice of either cross, as they age, develop abnormal serum autoantibodies and glomerulonephritis

Mohan et al. (*supra*) conducted study in which young female (SWR X NZB) F₁ mice were treated with three doses of 250 mg MR1, administered to three-month old mice every other day for three days. Animals were killed after they developed nephritis, defined as proteinuria of at least 300 µg/dl for two consecutive weeks. The untreated animals began to develop nephritis at six months, with 60% having nephritis by 10 months. The first of the MR1-treated animals developed nephritis only at 9 months, with only 40% of the treated animals showing nephritis at 12 months, when the study ended. The treatment also was associated with a prolonged decrease in serum levels of anti-single stranded DNA (anti-ssDNA) and anti-double stranded DNA (anti-dsDNA) autoantibodies: in 7 month old animals. either untreated or 4 months after the MR1 therapy. median anti-ssDNA was 4.5 U/ml or 0.6 U/ml respectively, and median anti-dsDNA was 2.2 U/ml or 0.4 U/ml respectively. The treatment had no effect on the serum levels of total IgG.

In the study by Early et al. (*supra*), female (NZB X NZW) F₁ mice were treated with the hamster anti-muCD40L mAb MR1 (Noelle et al., Proc. Natl. Acad. Sci. 89:6550, 1992). given i.p. at the substantial dose of 200 mg MR1 twice weekly from ages 4 to 10 months. The treatment commenced before the age at which any of the mice developed severe nephritis. as evidenced by significant proteinuria of at least 500 mg/dl. (The first of the ten untreated control mice to develop severe proteinuria did so at 4.5 months (Early et al., *supra* at 3161)). MR1 treatment substantially inhibited the development of nephritis in the treated animals, and prolonged their survival: while half of the untreated F₁ mice developed nephritis by 6 months, and all were dead by 10 months, fewer than half the MR1-treated animals had nephritis and only 35% had died by age 10 months, when the MR1 treatment ended. The MR1 treatment also caused the anti-DNA autoantibody liter to stabilize for several months after initiation of treatment, while in the untreated controls anti-ssDNA antibody titres rose to much higher levels during the same period.

While the results of these studies are informative. they are of limited relevance to use of analogous therapies in patients with spontaneous SLE for the following reasons. Most importantly, the mice in the above studies were treated with anti-CD40L mAb beginning before proteinuria was evident. Therefore, while the studies' results may suggest that anti-CD40L therapy might be able to prevent the development of nephritis when the therapy is initiated before symptomatic disease is present, the results do not address what might occur in the clinically relevant situation where the subject has established, symptomatic lupus. In addition, the model used by Early et al., the (NZB X NZW) F₁ mouse, may not have similar enough pathogenesis of nephritis to that of human SLE patients to be predictive for outcome of a therapeutic intervention. For example, the (NZB X NZW) F₁ mouse has high blood levels of retroviral protein, which along with serum autoantibodies is thought to contribute to the animals' nephritis. Since most human SLE patients do not have retroviral proteins in their blood, and the effects of anti-CD40L compounds on levels of retrovirus are unknown, the effects of anti-CD40L compounds on autoantibody-associated human lupus nephritis are not predictable from results of therapeutic intervention in the (NZB X NZW) F₁ mouse.

### Summary of the Invention

The invention provides a method for treating, reversing, stabilizing or inhibiting progression of nephritis in a patient with either immune complex disease or with symptomatic SLE, by administering a therapeutically effective amount of an anti-CD40L compound to the patient. By "symptomatic SLE", we mean that the patient has one or more of the following: proteinuria of over 150 mg/L; urinary protein totaling over 150 mg/day; or serum levels of anti-dsDNA antibodies which are higher than normal human levels. Active nephritis is often indicated by proteinuria of over 300 mg/L.

The anti-CD40L compound may be any compound that binds to CD40L on the surface of CD40L-expressing cells, such as activated T cells. In one embodiment, the compound is an anti-CD40L antibody, preferably a monoclonal antibody. The monoclonal antibody may be 5c8 (ATCC Accession No. HB 10916).

The anti-CD40L compound may be formulated in a therapeutic composition which includes a therapeutically-effective amount of the anti-CD40L compound and a pharmaceutically acceptable carrier. The therapeutic composition may also include a second therapeutically effective compound.

The invention also provides a method for improving renal function in a patient with immune complex disease, which includes administering a therapeutically effective amount of an anti-CD40L compound to the patient, then measuring protein levels in the patient's urine which are lower than urine protein levels measured before administration of the anti-CD40L compound.

The invention further provides a medical product which encompasses a therapeutic composition; the composition includes a therapeutically-effective amount of an anti-CD40L compound and a pharmaceutically acceptable carrier, sterilely packaged in a container with instructions for use in treating lupus nephritis or other immune complex diseases. In this composition, the anti-CD40L compound may be a monoclonal antibody, such as 5c8.

In another aspect, the invention provides a method of inhibiting the development or progression of nephritis in a kidney allograft within a patient with an immune complex disease. In this method, a therapeutically effective amount of an anti-CD40L compound is administered to the patient. This method may be useful in patients with any immune complex disease, including SLE and serum sickness. The anti-CD40L compound may be administered to the patient before the time of transplant, at time of transplant, following transplant, periodically following transplant of the allograft into the patient, or following more than one of these dosing regimes.

### Brief Description of the Drawings

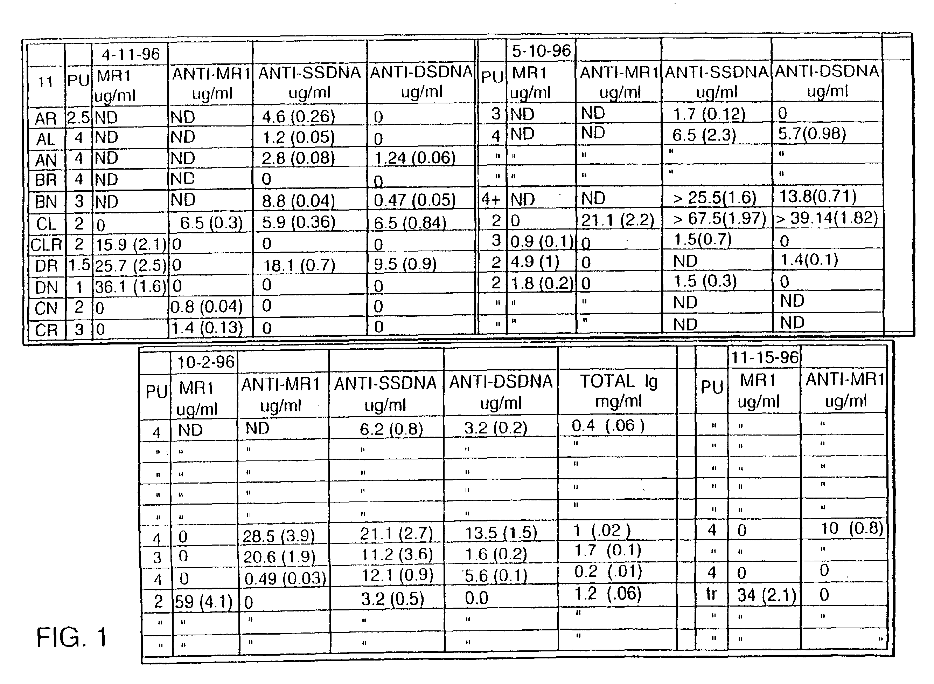

Fig. 1 is a chart of the changes with time in several measured characteristics of blood and urine from control and treated (SWR X NZB) F₁ mice in Experiment II. The anti-CD40L mAb MR1, at 500 ug/animal i.p., was administered once when the mice were 4 months old. again at 7 month of age, again at 9 months, and then at monthly intervals. Each of the upper five rows of the chart, marked AR-BN, contains data from a single control animal, and each of the lower six rows, marked CL-CR , contains data from a single treated animal. This study began when the animals were 4 months of age, in February 1996. The vertical double lines separate 4 groups of data, each data group providing the measurements for urine and blood samples collected on the date listed above the data. Proteinuria (PU) levels are indicated from trace to level 4. Level 1 correlates with urine albumin of 30 mg/dl albumin, level 2 with 100 mg/dl, level 3 with 300 mg/dl, and level 4 with over 2000 mg/dl. Levels of anti-MR1 antibodies (provided in column labeled "anti-MR1"), anti-ssDNA antibodies and anti-dsDNA antibodies are given in µg/ml blood. Where appropriate, values are given as mean and standard deviation of several samples, in the form mean(S.D.). A dash indicates that a sample was not collected, typically because the animal had died. ND refers to "not done."

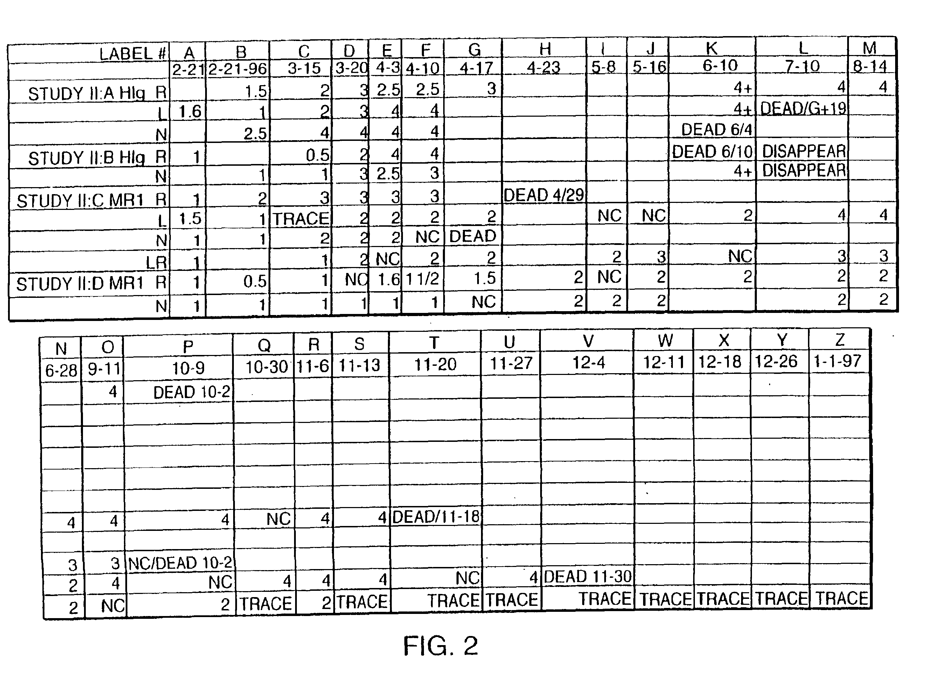

Fig. 2 is a chart of proteinuria measurements of the Experiment II animals over time. The first column provides the animal numbers as in Fig. 1. The columns are headed with the dates of sample collection. NC means "not collected."

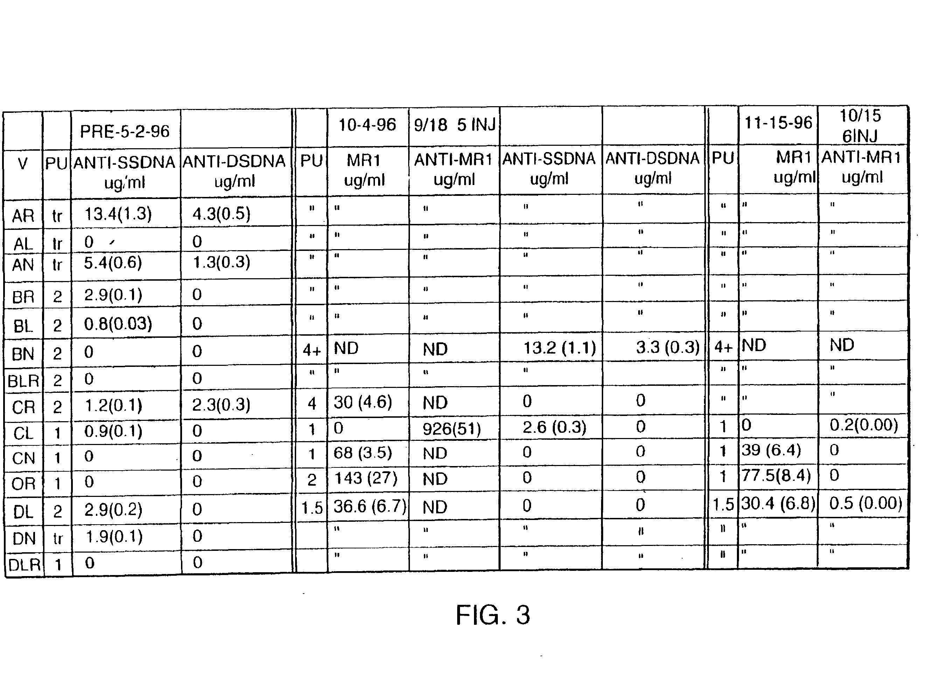

Fig. 3 is a chart of blood and urine characteristics with time in Experiment V control and untreated mice, which started treatment at 4.5 months of age. MR1 was administered to treated animals once at 500 ug/animal i.p. when the mice were 4.5 months old, and then as monthly injections of 500 ug, i.p. Each of the upper seven rows of the chart, marked AR-BLR, contains data from a single control animal, and each of the lower seven rows. marked CR -CLR, contains data from a single treated animal. This study began when the animals were 4.5 months of age, in May 1996. Other descriptions of the figure are the same as those of Fig. 1.

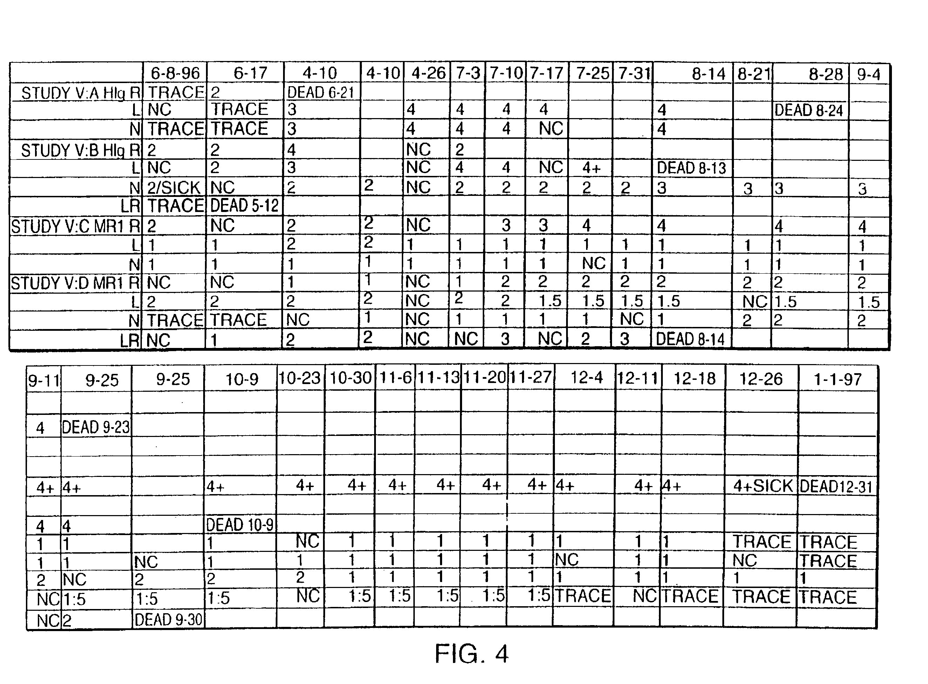

Fig. 4 is a chart of proteinuria measurements of the Experiment V animals over time. Animal numbers are as described for Fig. 3. Other descriptions of the figure are the same as those of Fig. 2.

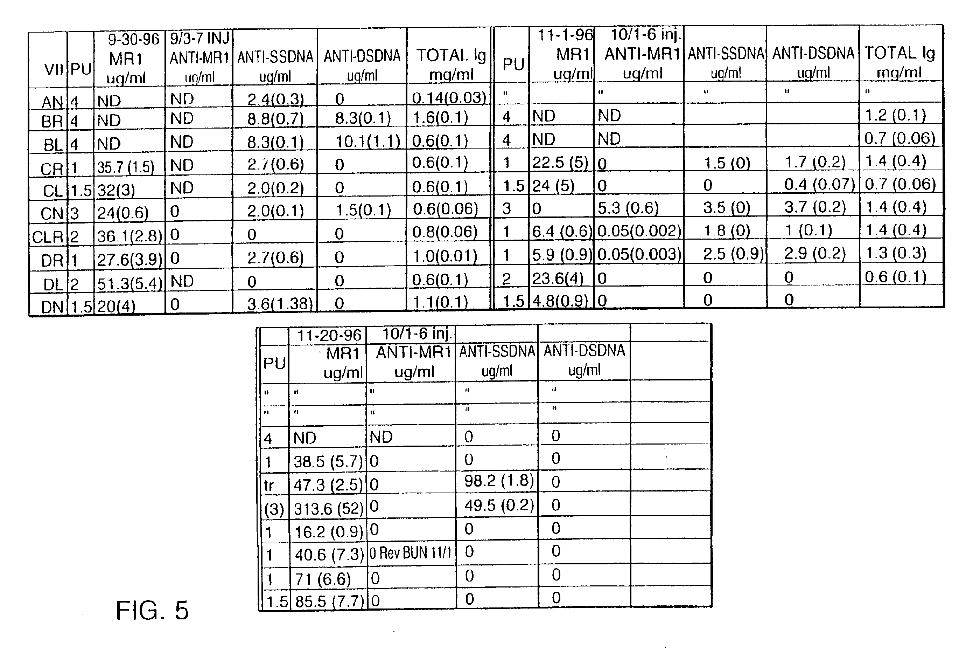

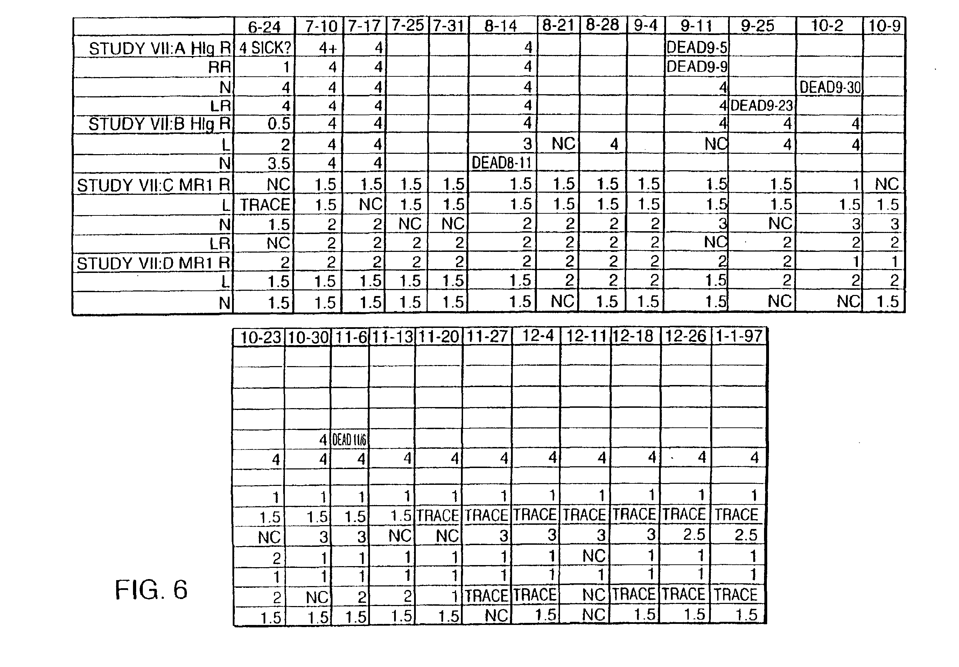

Fig. 5 is a chart of chart of blood and urine characteristics with time in Experiment VII control and untreated mice, which started treatment at 5.5 months of age. MR 1 was administered to treated animals once weekly at 500 ug/animal i.p. for six weeks, followed by monthly injections of 500 ug, i.p. Each of the upper three rows of the chart, marked AN-BL, contains data from a single control animal (as noted in Fig. 6, some control animals had died before the data for Fig. 5 was collected), and each of the lower seven rows, marked CR-DN , contains data from a single treated animal. This study began when the animals were 5.5 months of age, in June 1996. Other descriptions of the figure are the same as those of Fig. 1.
Fig. 6 is a chart of proteinuria measurements of the Experiment VII animals over time. Each of the upper seven rows of the chart, marked AR-BN, contains data from a single control animal, and each of the lower seven rows, marked CR-DN , contains data from a single treated animal. Other descriptions of the figure are the same as those of Fig. 2.

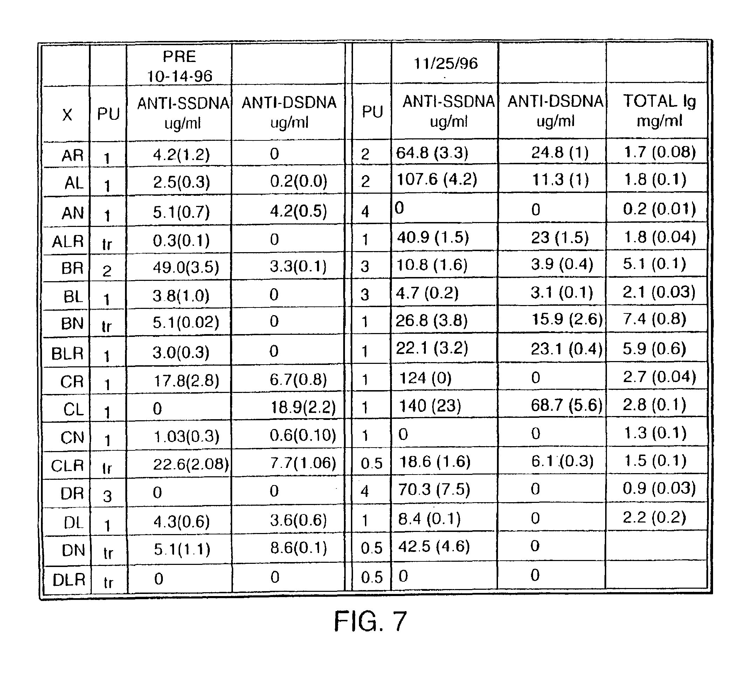

Fig. 7 is a chart of blood and urine characteristics with time in Experiment X control and untreated mice, which started treatment at 5.5 months of age. MR1 was administered to treated animals once weekly at 500 ug/animal i.p. for four weeks, followed by monthly injections of 200 ug, i.p. Each of the upper eight rows of the chart, marked AR-BLR, contains data from a single control animal, and each of the lower eight rows, marked CR-DLR , contains data from a single treated animal. This study began when the animals were 5.5 months of age, in October 1996. Other descriptions of the figure are the same as those of Fig. 1.

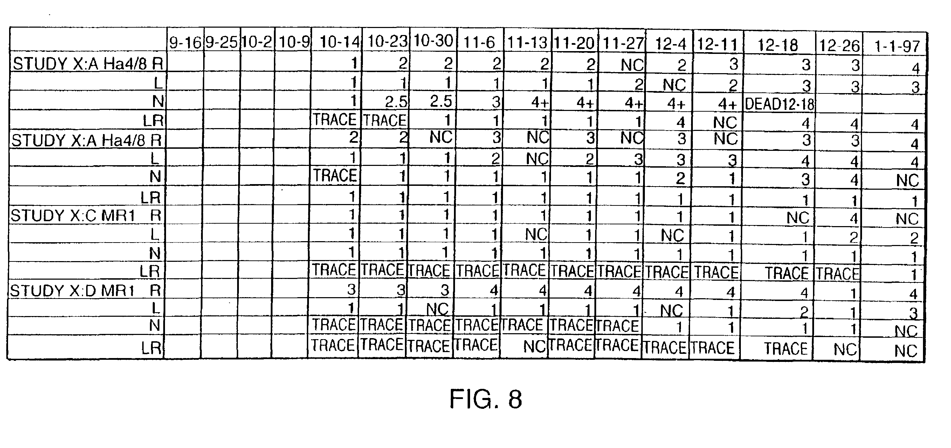

Fig. 8 is a chart of proteinuria measurements of the Experiment X animals over time. The first column provides the animal numbers as in Fig. 7. Other descriptions of the figure are the same as those of Fig. 2.

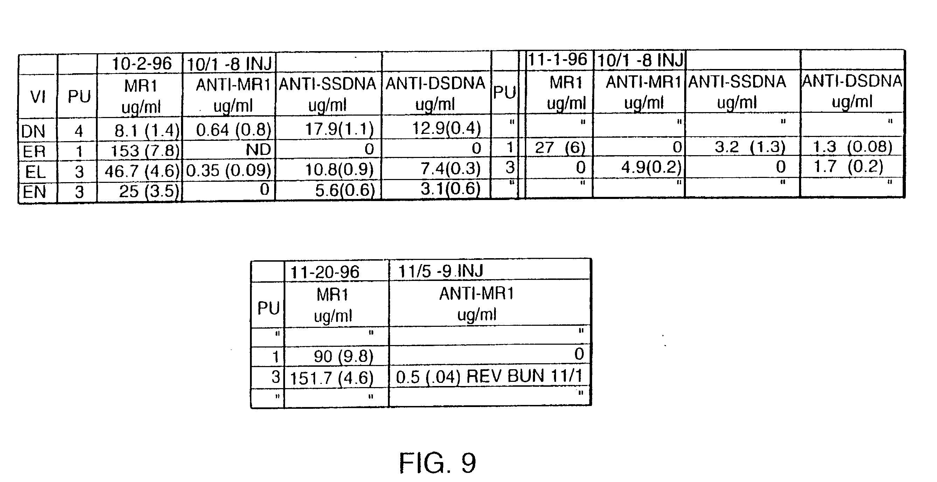

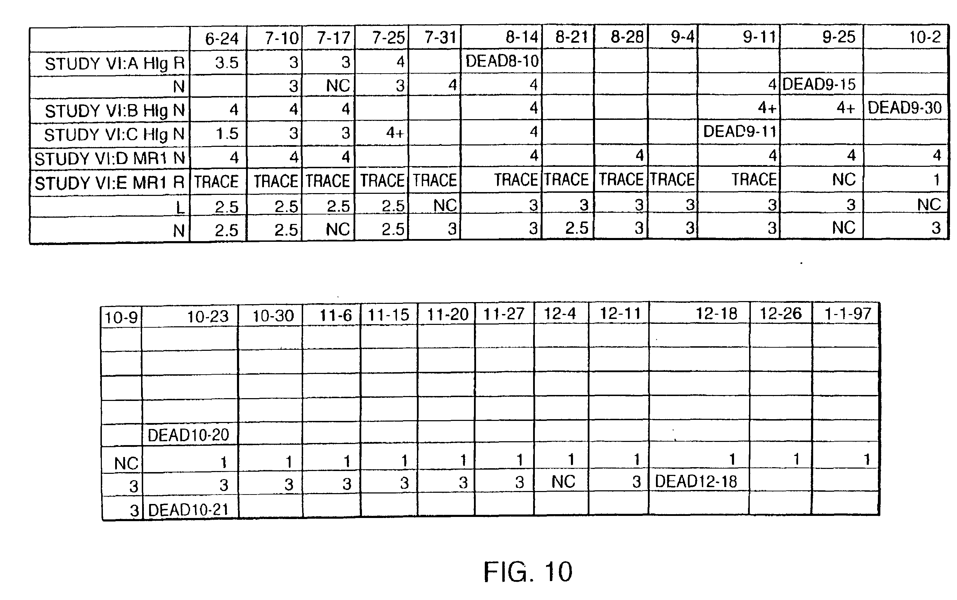

Fig. 9 is a chart of blood and urine characteristics with time in Experiment VI control and untreated mice, which started treatment at 7 months of age. MR1 was administered to 4 treated animals once weekly at 500 ug/animal i.p. for six weeks, followed by monthly injections of 500 ug, i.p. Each of the lower four rows, marked DN-EN, contains data from a single treated animal. At the time of first data collection for this chart, all control animals had died, as noted Fig. 10. This study began when the animals were 7 months of age, in June 1996. Other descriptions of the figure are the same as those of Fig. 1.
Fig. 10 is a chart of proteinuria measurements of the Experiment VI animals over time. Each of the upper four rows of the chart, marked AR-CN, contains data from a single control animal, and each of the lower four rows, marked DN-EN, contains data from a single treated animal. Other descriptions of the figure are the same as those of Fig. 2.

### Detailed Description of the Invention

The method of the invention involves treating, reversing or stabilizing nephritis in patients with either immune complex disease or with established SLE. The patients are treated with a compound that blocks the interaction of CD40L on T cells with CD40 on B cells. This is thought to inhibit the production of pathologic antibodies, which in turn reduces the levels of pathogenic autoantibodies associated with developing and exacerbating nephritis associated with immune complexes.

### Compounds

Therapeutic compounds useful for the methods of the invention include any compound that blocks the interaction of CD40 on B cells with CD40L expressed on the surface of activated T cells. Anti-CD40L compounds specifically contemplated include polyclonal antibodies and monoclonal antibodies (mAbs), as well as antibody derivatives such as chimeric molecules, humanized molecules, molecules with reduced effector functions, bispecific molecules, and conjugates of antibodies. In a preferred embodiment, the antibody is 5c8, as described in U.S. Patent 5,474,771, the specification of which is hereby incorporated by reference. Other known antibodies against 5c8 antigen include antibodies ImxM90, ImxM91 and ImxM92 (obtained from Immunex), an anti-CD40L mAb commercially available from Ancell (clone 24-31, catalog # 353-020, Baypon, MN), and an anti-CD40L mAb commercially available from Genzyme (Cambridge, MA, catalog # 80-3703-01). Also commercially available is an anti-CD40L mAb from PharMingen (San Diego, catalog #33580D). Numerous additional anti-CD40L antibodies have been produced and
characterized (see, e.g., WO 96/23071 of Bristol-Myers Squibb, the specification of which is hereby incorporated by reference).

The invention also includes anti-CD40L molecules of other types, such as complete Fab fragments, F(ab') compounds, V_{H} regions, Fᵥ regions, single chain antibodies (see, e.g., WO 96/23071), polypeptides, fusion constructs of polypeptides, fusions of CD40 (such as CD40Ig, as in Hollenbaugh et al., J. Immunol. Meth. 188:1-7, 1995, which is hereby incorporated by reference), and small molecule compounds such as small semi-peptidic compounds or non-peptide compounds, all capable of blocking the CD40-CD40L interaction. Procedures for designing, screening and optimizing small molecules are provided in the patent application PCT/US96/10664, filed June 21, 1996, the specification of which is hereby incorporated by reference.

Various forms of antibodies may also be produced using standard recombinant DNA techniques (Winter and Milstein, Nature 349: 293-99, 1991). For example, "chimeric" antibodies may be constructed, in which the antigen binding domain from an animal antibody is linked to a human constant domain ( an antibody derived initially from a nonhuman mammal in which recombinant DNA technology has been used to replace all or part of the hinge and constant regions of the heavy chain and/or the constant region of the light chain, with corresponding regions from a human immunoglobin light chain or heavy chain) (see, e.g., Cabilly et al., United States patent 4,816,567; Morrison et al., Proc. Natl. Acad. Sci. 81: 6851-55, 1984). Chimeric antibodies reduce the immunogenic responses elicited by animal antibodies when used in human clinical treatments.

In addition, recombinant "humanized" antibodies may be synthesized. Humanized antibodies are antibodies initially derived from a nonhuman mammal in which recombinant DNA technology has been used to substitute some or all of the amino acids not required for antigen binding with amino acids from corresponding regions of a human immunoglobin light or heavy chain (chimeras comprising mostly human IgG sequences into which the regions responsible for specific antigen-binding have been inserted)(see, e.g., PCT patent application WO 94/04679). Animals are immunized with the desired antigen, the corresponding antibodies are isolated and the portion of the variable region sequences responsible for specific antigen binding are removed. The animal-derived antigen binding regions are then cloned into the appropriate position of the human antibody genes in which the antigen binding regions have been deleted. Humanized antibodies minimize the use of heterologous (inter-species) sequences in human antibodies and are less likely to elicit immune responses in the treated subject.

Also useful in the methods and compositions of this invention are primate or primatized antibodies.

Antibody fragments and univalent antibodies may also be used in the methods and compositions of this invention. Univalent antibodies comprise a heavy chain/light chain dimer bound to the Fc (or stem) region of a second heavy chain. "Fab region" refers to those portions of the chains which are roughly equivalent, or analogous, to the sequences which comprise the Y branch portions of the heavy chain and to the light chain in its entirety, and which collectively (in aggregates) have been shown to exhibit antibody activity. A Fab protein includes aggregates of one heavy and one light chain (commonly known as Fab'), as well as tetramers which correspond to the two branch segments of the antibody Y, (commonly known as F(ab)₂), whether any of the above are covalently or non-covalently aggregated, so long as the aggregation is capable of selectively reacting with a particular antigen or antigen family.

In addition, standard recombinant DNA techniques can be used to alter the binding affinities of recombinant antibodies with their antigens by altering amino acid residues in the vicinity of the antigen binding sites. The antigen binding affinity of a humanized antibody may be increased by mutagenesis based on molecular modeling (Queen et al., Proc. Natl. Acad. Sci. 86:10029-33, 1989; PCT patent application WO 94/04679). It may be desirable to increase or to decrease the affinity of the antibodies for CD40L, depending on the targeted tissue type or the particular treatment schedule envisioned. This may be done utilizing phage display technology (see, e.g.. Winter et al., Ann. Rev. Immunol. 12:433-455, 1994; and Schier et al., J. Mol. Biol. 255:28-43, 1996, which are hereby incorporated by reference). For example, it may be advantageous to treat a patient with constant levels of antibodies with reduced affinity for CD40L for semi-prophylactic treatments. Likewise, antibodies with increased affinity for CD40L may be advantageous for short-term treatments.

### Subjects

The subjects for which the methods of the invention are intended have immune complex disease. These diseases are characterized by the presence of circulating immunoglobulins and immune complexes in the blood. One class of immune complex diseases is called serum sickness, and can be caused by immune reaction to an exogenous antigen, such as an infectious agent, a drug, foreign antisera, or blood products. Immune complex disease can also occur when a patient makes "autoantibodies", which are antibodies against a component of the patient's own tissues. Examples of such immune complex autoimmune diseases are SLE, rheumatoid arthritis, Goodpasture's syndrome, Wegener's granulomatosis, microscopic polyarteritis, polyarteritis nodosa, Churg-Strauss syndrome, and diverse other forms of vasculitis. Nephritis related to immune-associated conditions which do not fall into the above categories may also be treated with the methods of the invention; conditions in this category include Henoch-Schönlein purpura, essential (mixed) cryoimmunoglobinemia. ANCA-associated glomerulonephritis, and monoclonal gammopathies such as multiple myeloma, benign monoclonal gammopathies and Waldenström's macroglobinemia.

The term "patient" is taken to mean any mammalian patient to which anti-CD40L compounds may be administered. Patients specifically intended for treatment with the method of the invention include humans, as well as nonhuman primates. sheep, horses, cattle, goals. pigs, dogs, cats, rabbits, guinea pigs, hamsters. gerbils, rats and mice, as well as the organs, tumors, and cells derived or originating from these hosts.

### Routes of Administration

The compounds of the invention may be administered in any manner which is medically acceptable. This may include injections, by parenteral routes such as intravenous, intravascular, intraarterial, subcutaneous, intramuscular, intratumor, intraperitoneal, intraventricular, intraepidural, or others as well as oral, nasal, ophthalmic, rectal, or topical. Sustained release administration is also specifically included in the invention, by such means as depot injections. Some forms of anti-CD40L compounds may be suitable for oral administration, and could be formulated as suspensions or pills.

### Dosages and Frequency of Treatment

The amount of and frequency of dosing for any particular compound to be administered to a patient for a given immune complex disease is a judgment made by the patient's physician, based on a number of factors. The general dosage is established by preclinical and clinical trials, which involve extensive experiments to determine the beneficial and deleterious effects on the patient of different dosages of the compound. Even after such recommendations are made, the physician will often vary these dosages for different patients based on a variety of considerations, such as a patient's age, medical status, weight, sex, and concurrent treatment with other pharmaceuticals. Determining the optimal dosage for each anti-CD40L compound used to treat lupus nephritis is a routine matter for those of skill in the pharmaceutical and medical arts.

Various regimens may be used for treatment of lupus or other immune complex diseases according to this invention. Generally, the frequency of dosing would be determined by the attending physician, and might be either as a single dose, or repeated daily, at intervals of 2-6 days, weekly, biweekly, or monthly.

To exemplify dosing considerations for an anti-CD40L compound, the following examples of administration strategies are given for an anti-CD40L mAb. The dosing amounts could easily be adjusted for other types of anti-CD40L compounds. In general. single dosages of between about 0.05 and about 50 mg/kg patient body weight are contemplated, with dosages most frequently in the 1-20 mg/kg range. For acute treatment, an effective dose of antibodies ranges from about 1 mg/kg body weight to about 20 mg/kg body weight, administered daily for a period of about 1 to 5 days, preferably by bolus intravenous administration. The same dosage and dosing schedule may be used in the load phase of a load-maintenance regimen, with the maintenance phase involving intravenous or intramuscular administration of antibodies in a range of about 0.1 mg/kg body weight to about 20 mg/kg body weight, for a treatment period of anywhere from weekly to 3 month intervals. Chronic treatment may also be carried out by a maintenance regimen, in which antibodies are administered by intravenous or intramuscular route, in a range of about 0.1 mg/kg body weight to about 20 mg/kg body weight, over a period anywhere between about weekly to 3 month intervals. The antibodies may also be administered in a range of about 0.2 mg/kg body weight to about 10 mg/kg body weight. In addition, chronic treatment may be effected by an intermittent bolus intravenous regimen, in which between about 1.0 mg/kg body weight and about 100 mg/kg body weight of antibodies are administered, for anywhere from monthly to 6 month intervals between treatments. For all except the intermittent bolus regimen, administration may also be by oral, pulmonary, nasal or subcutaneous routes.

Generally, therapy is commenced with low doses of antibodies. For example, an initial dose of antibodies is administered to the patient by, for example, injection or infusion. That initial dose should contain between about 1.0 mg and 30 mg of antibodies per day for a 70 kg patient. For repeated administrations over several days, dosages may be administered on successive days, every two to six days, once a week, every two to four weeks or once a month, until a desired suppression of disease symptoms is observed. However, other dosage regimens are also useful. When the symptoms have been alleviated to the desired level, treatment may cease. Patients may, however, require intermittent treatment on a long term basis upon recurrence of disease symptoms.

According to an alternate embodiment of this invention for treatment of lupus, the effectiveness of the antibodies may be increased by administration serially or in combination with conventional anti-lupus therapeutic agents or drugs such as, for example, salicylates, corticosteroids or immunosuppressants. Alternatively, the antibodies may be conjugated to a conventional agent. This advantageously permits the administration of the conventional agent in an amount less than the conventional dosage, for example, less than about 50% of the conventional dosage, when the agent is administered as monotherapy. Accordingly, the occurrence of many side effects associated with that agent might be avoided.

Combination therapies according to this invention for treatment of lupus include the use of anti-CD40L antibodies together with agents targeted at B cells, such as anti-CD19, anti-CD28 or anti-CD20 antibody (unconjugated or radiolabeled), IL-14 antagonists, LJP394 (LaJolla Pharmaceuticals receptor blocker), IR-1116 (Takeda small molecule) and anti-Ig idiotype monoclonal antibodies. Alternatively, the combinations may include T cell/B cell targeted agents, such as CTLA4Ig, IL-2 antagonists, IL-4 antagonists, IL-6 antagonists, receptor antagonists, anti-B7 monoclonal antibodies, TNF, LFAI/ICAM antagonists, VLA4/VCAM antagonists, brequinar and IL-2 toxin conjugates (e.g., DAB), prednisone, cyclophosphamide, and other immunosuppressants. Combinations may also include T cell targeted agents, such as CD4 antagonists, CD2 antagonists and IL-12.

Combination therapies for treatment of a patient with a non-lupus immune complex disease might involve administration of an anti-CD40L compound as well as an agent which would typically be administered for the particular immune complex disease in question.

Once improvement of the patient's condition has occurred, a maintenance dose of anti-CD40L antibodies, alone or in combination with a conventional anti-lupus agent is administered, if necessary. Subsequently, the dosage or the frequency of administration, or both, may be reduced, as a function of the symptoms, to a level at which the improved condition is retained. When the symptoms have been alleviated to the desired level, treatment might cease. In other instances, as determined by a patients physician, occasional treatment might be administered, for example at intervals of four weeks or more. Patients may, however, require intermittent treatment on a long-term basis upon any recurrence of disease symptoms.

### Formulation

An anti-CD40L compound used in the methods of the invention is administered in a pharmaceutically-effective amount, which is an amount which produces a medically beneficial effect on the kidney of a patient with an immune complex disease, particularly SLE. Medically beneficial effects would include preventing deterioration or causing improvement in renal function or health. Renal function and health may be monitored with one or more laboratory tests which measure the concentrations of relevant substances in blood or urine. other urine characteristics, or the rate of clearance of various substances from the blood into the urine. The parameters measured by these tests, either individually or in combination, can be used by a physician to assess renal function or damage. Examples of such parameters include the blood concentration of urea, creatinine or protein; the urine concentration of protein or of various blood cells such as erythrocytes or leucocytes; urine specific gravity; amount of urine; the clearance rates of inulin, creatinine, urea or ρ-aminohippuric acid; and the presence of hypertension or edema. Medically beneficial effects would also include the diminution of autoantibodies, such as anti-dsDNA antibodies in the serum of lupus patients.

An anti-CD40L compound of the invention is administered to a patient in a pharmaceutically acceptable composition, which may include a pharmaceutically-acceptable carrier. Such a carrier is relatively non-toxic and innocuous to a patient at concentrations consistent with effective activity of the anti-CD40L compound or other active ingredients, so that any side effects ascribable to the carrier do not vitiate the beneficial effects of the active ingredients of the composition. The composition may include other compatible substances; compatible, as used herein, means that the components of the pharmaceutical composition are capable of being commingled with the anti-CD40L compound, and with each other, in a manner such that there is no interaction which would substantially reduce the therapeutic efficacy of the pharmaceutical. Nasal spray formulations comprise purified aqueous solutions of the active compound with preservative agents and isotonic agents. Such formulations are preferably adjusted to a pH and isotonic state compatible with the nasal mucous membranes. Formulations of the present invention suitable for oral administration may be presented as discrete units such as capsules, cachets, tablets, pills or lozenges, each containing a predetermined amount of the potentiating anti-CD40L compound as a powder or granules; as liposomes; or as a suspension in an aqueous liquor or non-aqueous liquid such as a syrup, an elixir, an emulsion or a draught.

The compositions of the invention may be provided in containers suitable for maintaining sterility, protecting the activity of the active ingredients during proper distribution and storage, and providing convenient and effective accessibility of the composition for administration to a patient. For an injectable formulation of an anti-CD40L compound, the composition might be supplied in a stoppered vial suitable for withdrawal of the contents using a needle and syringe. The vial would be intended for either single use or multiple uses. The composition might also be supplied as a prefilled syringe. In some instances, the contents would be supplied in liquid formulation, while in others they would be supplied in a dry or lyophilized state, which would require reconstitution with a standard or a supplied diluent to a liquid state. Where the compound is supplied as a liquid for intravenous administration, it might be provided in a sterile bag or container suitable for connection to an intravenous administration line or catheter. In instances where the anti-CD40L compound is orally administered in tablet or pill form, the compound might be supplied in a bottle with a removable cover. The containers may be labeled with information such as the type of compound, the name of the manufacturer or distributor, the indication, the suggested dosage, instructions for proper storage, or instructions for administration.

### Use of Anti-CD40L Compounds to Treat Lupus Nephritis in Nonhuman Subjects

We tested the effects of the hamster anti-muCD40L mAb MR1 on the course of nephritis in the female (SWR X NZB) F₁ mouse, in several studies as described below. Control animals were injected either with Syrian hamster polyclonal Ig or with Ha4/8, an Armenian hamster mAb directed against KLH. Proteinuria levels are indicated from trace to level 4. Level 1 correlates with urine albumin of 30 mg/dl albumin, level 2 with 100 mg/dl, level 3 with 300 mg/dl, and level 4 with over 2000 mg/dl. A level of 2 was considered to indicate moderate nephritis, with 2.5 and greater indicating severe nephritis.

If untreated, or if treated with the nonspecific hamster immunoglobulins administered to control animals, the mice normally die by 12 months of age. While the onset of proteinuria in untreated animals is variable, most have mild to moderate proteinuria by 3 months of age ; the proteinuria tends to increase with age. By about 5 months of age, all control animals typically have detectable anti-dsDNA antibodies, and most have detectable anti-ssDNA antibodies; this contrasts with the complete lack of detectable levels of these antibodies in normal mice, such as the female SWR parents of the (SWR X NZB) F₁ mice.

### Experiment II: Treatment begun at 4 months (Figs. 1 and 2)

MR1 treatment was initiated when the mice were 4 months of age. MR1 was administered to treated animals once at 500 ug/animal i.p. when the mice were 4 months old, once at 7 months of age, and once at 9 months followed by once-monthly injections. After 4 months of treatment, 4 of the 5 control animals had died, but four of the six treated animals were yet alive. Three of these four previously surviving treated mice died, one each at 12, 13 and 13.5 months. One still survives, and is now 15 months old, an extraordinary longevity for mice of this cross. Of great interest, the surviving animal (mouse II:DN on Figure 2) had moderate nephritis (2+ proteinuria) from ages 8 to 13 months, which has decreased to only trace levels of proteinuria for the last two months. This is the first demonstration of a functional reversal of nephritis in a mouse of this strain.

### Experiment V: Treatment begun at 4.5 months (Figs. 3 and 4)

MR1 treatment was initiated when the mice were 4.5 months of age. MR1 was administered to treated animals once at 500 ug/animal i.p. when the mice were 4.5 months old, and then as monthly injections of 500 ug, i.p. After 4.5 months, 6 of the 7 control animals had died, but six of the seven treated animals survived. After 8 months of treatment, all controls were dead, but only three of the seven treated mice had died. As shown in Fig. 4, four of the seven MR 1-treated animals had their nephritis reversed as shown by sustained lowered proteinuria levels. These four animals are still alive at age 12.5 months.

### Experiment VII: Treatment begun at 5.5 months (Figs. 5 and 6)

MR1 treatment was initiated when the mice were 5.5 months of age. MR1 at 500 µg/animal i.p. was administered to treated animals once weekly for six weeks, followed by monthly injections. After 5 months of treatment, at age 10.5 months, 6 of the 7 control animals had died; all of the 7 treated animals are still alive at age 12 months. The following values were measured in the animals which still survived at 8.5 months, after 3.5 months of treatment.

| | anti-SS-DNA | anti-DS DNA | PU |
|---|---|---|---|
| control | 2.4 | 0 | 4 |
| | 8.8 | 6.3 | 4 |
| | 6.3 | 10.1 | 4 |
| Mean (Std. Dev.) | 5.8 (2.6) | 5.4 (4.1) | 4 (0) |
| | | | |
| MR1 | 2.7 | 0 | 1 |
| | 2.0 | 0 | 1.5 |
| | 2.0 | 1.5 | 3 |
| | 0 | 0 | 2 |
| | 2.7 | 0 | 1 |
| | 0 | 0 | 2 |
| | 3.5 | 0 | 1.5 |
| Mean (Std. Dev.) | 1.8 (1.2) | 0.2 (0.5) | 1.7 ( ) |

### Experiment X: Less intensive treatment, begun at 5.5 months (Figs. 7 and 8)

MR1 treatment was initiated when the mice were 5.5 months of age. MR1 was administered to treated animals once weekly at 500 ug/animal i.p. for four weeks, followed by monthly injections of 200 µg, i.p. Of the 16 mice in the study (8 each in control and treated groups), now 8.5 months of age, only one mouse has died, a control animal at 7.5 months. As shown in Fig. 8, seven of the eight control animals had proteinuria which steadily increased to high levels, averaging +3.4 for the 7 surviving control mice. All but one of the eight MR1- treated mice have maintained low proteinuria, which currently averages +2 for the 8 treated mice. As shown in Fig. 7, six of the treated animals, but only one of the controls. have no detectable anti-dsDNA antibodies.

### Experiment VI: Treatment begun at 7 months (Figs. 9 and 10)

MR1 treatment was initiated when the mice were seven months of age. MR1 was administered to 4 treated animals once weekly at 500 ug/animal i.p. for six weeks, followed by monthly injections of 500 ug, i.p. By age 10 months, all 4 control animals had died. While 2 of the treated mice died at age 11 months, and a third at 13 months, one of the four treated animals remains alive currently at 14 months of age, after 7 months of treatment. The surviving treated animal (number VI:ER) currently has level 1 proteinuria, and detectable anti-dsDNA and anti-ssDNA antibodies.

These experiments show that treatment of (SWR X NZB) F₁ mice with anti-CD40L mAb prolongs survival as compared to control animals, and slows development of nephritis as indicated by proteinuria levels. In some animals, the treatment reverses nephritis, as shown by a reduction in proteinuria levels. Of 32 treated animals, 11 had urine protein levels which decreased with anti-CD40L mAb therapy; none of the control animals had similar reductions. Of 24 treated animals in which serum blood urea nitrogen (BUN) was measured, 3 had decreases in BUN levels after treatment, which was not observed in any control animal. In addition, MR treatment results in a reduced serum concentration of anti-DS and anti-SS DNA autoantibodies, which are normally produced in untreated animals of this type.

Although the foregoing invention has been described in some detail by way of illustration and example for purposes of clarity of understanding, it will be obvious to one skilled in the art that certain changes and modifications may be practiced within the scope of the invention, as limited only by the scope of the appended claims.

## Claims

1. A method of inhibiting the progression of or treating nephritis in a patient with immune complex disease, comprising administering a therapeutically effective amount of an anti-CD40L compound to the patient.

2. The method of claim 1, wherein the immune complex disease is symptomatic lupus.

3. The method of claim 2, wherein the patient has serum levels of anti-dsDNA antibodies which are higher than normal human levels.

4. The method of claim 1. wherein the patient has proteinuria of over 150 mg/L.

5. The method of claim 1, wherein the patient has urinary protein totaling over 150 mg/day.

6. The method of claim I. wherein the anti-CD40L compound is an anti-CD40L antibody.

7. The method of claim 6. wherein the antibody is a monoclonal antibody.

8. The method of claim 7, wherein the monoclonal antibody is 5c8.

9. The method of claim 1, wherein the anti-CD40L compound is formulated in a therapeutic composition comprising a therapeutically-effective amount of the anti-CD40L compound and a pharmaceutically acceptable carrier.

10. The method of claim 9, wherein the therapeutic composition further comprises a second therapeutically effective compound.

11. A method of improving renal function in a patient with immune complex disease, comprising administering a therapeutically effective amount of an anti-CD40L compound to the patient and thereafter measuring protein levels in the patient's urine which are lower than urine protein levels measured before administration of the anti-CD40L compound.

12. A medical product comprising a therapeutic composition comprising a therapeutically-effective amount of an anti-CD40L compound and a pharmaceutically acceptable carrier, sterilely packaged in a container with instructions for use in treating an immune complex disease.

13. The product of claim 12, wherein the anti-CD40L compound is a monoclonal antibody.

14. A method of inhibiting the development or progression of nephritis in a kidney allograft within a patient with an immune complex disease, comprising administering a therapeutically effective amount of an anti-CD40L compound to the patient.

15. The method of claim 14, wherein the disease is SLE.

16. The method of claim 14, wherein the anti-CD40L compound is administered to the patient periodically following transplant of the allograft into the patient.
